# EUROPÄISCHE PATENTANMELDUNG

(11) **EP 1 586 266 A1**
(43) Veröffentlichungstag der Anmeldung: **19.10.2005**
(21) Anmeldenummer: 04009088.8
(22) Anmeldetag: 16.04.2004
(51) Int. Cl.: A61B 1/24

(54) **Betätigungsantrieb für eine Optik insbesondere im Bereich Intraoralkamera**

(71) Anmelder: Orangedental GmbH & Co. KG, 88400 Biberach (DE)
(72) Erfinder: Piernitzki, Bernhard, 19089 Demen (DE)
(74) Vertreter: Zipse + Habersack

(57) **Zusammenfassung**

Betätigungsantrieb für eine Optik, insbesondere im Bereich einer Intraoralkamera mit zumindest zwei an der Optik (3) angreifenden, gegeneinander arbeitenden Memorydrähten (1, 2) und mit einer Einrichtung (V1, V2) zum wechselseitigen Erwärmen dieser Memorydrähte.

## Beschreibung

Die Erfindung betrifft einen Betätigungsantrieb für eine Optik insbesondere im Bereich Intraoralkamera.

Bei Intraoralkameras, in der Endoskopie oder allgemein bei Kameras stellt sich das Problem für einen einfachen und klein bauenden mechanischen Antrieb einer variablen Blende und/oder für eine optische Fokussierung. Mit der vorliegenden Erfindung soll ein solcher Betätigungsantrieb für eine Optik insbesondere im Bereich Intraoralkamera geschaffen werden.

Die gestellte Aufgabe wird mit einem Betätigungsantrieb gemäß Anspruch 1 gelöst. Weiterbildungen der Erfindung sind in den Unteransprüchen beschrieben.

Die Idee der Erfindung ist, den Antrieb mit zwei gegeneinander arbeitenden Memorydrähten wie Nitinol- bzw. Flexinoldrähten auszuführen, die zum Beispiel an der variablen Blende einer Intraoralkamera angreifen, welche Blende das gleiche Prinzip hat wie in einem klassischen Fotoapparat und durch eine Drehbewegung (ca. 90°) geöffnet und geschlossen wird. Die Drähte verkürzen sich, wenn sie einer bestimmten hohen Temperatur ausgesetzt werden und verlängern sich, wenn sie wieder abkühlen. Die zur Zeit marktüblichen Memorydrähte verkürzen sich um beispielsweise 4 % bei ca. 70° C und verlängern sich wieder bei ca. 50° C. Ein Draht von 0,1 mm Durchmesser entwickelt bei der Verkürzung eine Kraft von etwa 15 N. Die Erhitzung der Drähte wird dadurch erreicht, indem sie von einem bestimmten Gleichstrom durchflossen werden.

Mit dem gleichen Prinzip kann auch eine optische Fokussierung betätigt werden.

Anstelle von zwei gegeneinander arbeitenden Memorydrähten kann gegebenenfalls ein einseitig an der Optik angreifender Memorydraht genügen. In diesem Fall ist ein elastisches Rückstellelement wie z.B. eine Ringfeder aus Stahl oder Gummi zum Rückstellen der Optik bei nachlassender Erwärmung des Memorydrahtes vorzusehen. In einem solchen Fall kann die Idee der Erfindung auch mit einem Draht verwirklicht werden, der sich bei Erwärmung dehnt. Das elastische Element öffnet dann z.B. die Blende, soweit der erwärmte und gedehnte Draht dies zulässt.

Die Erfindung wird nachfolgend anhand beigefügter Zeichnungen näher erläutert. Es zeigen
- Figur 1: eine Prinzipskizze und
- Figur 2: eine Ausführungsvariante des Betätigungsantriebs nach der Erfindung.

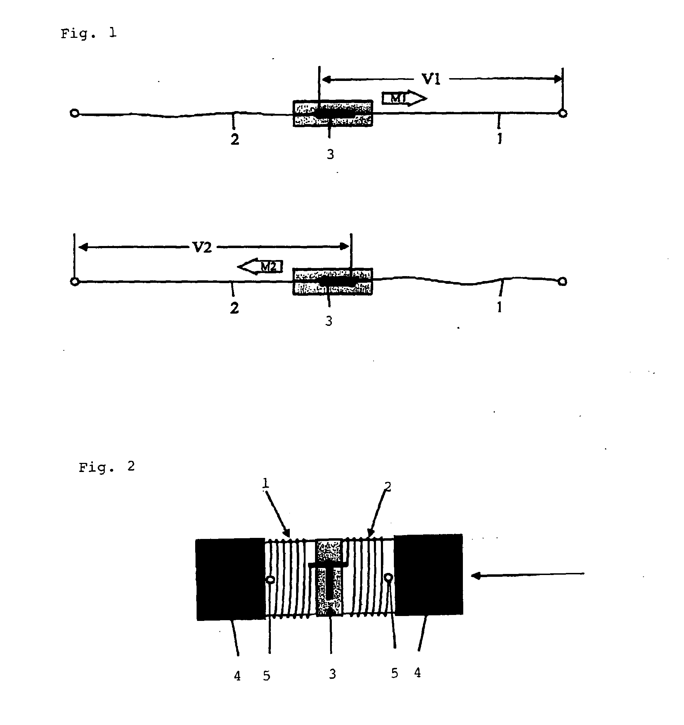

Die Figur 1 zeigt im Rechteck in der Mitte eine Blende 3 in Seitenansicht, an deren Betätigungsarm gegeneinander arbeitende Memorydrähte 1 und 2 befestigt sind. Der Memorydraht 2 ist so weit entspannt, dass der Blendensteller (Betätigungsarm der Blende) bis zum Anschlag nach rechts bewegt werden kann. Wird nun am Memorydraht 1 die Spannung V1 angelegt, erhitzt und verkürzt sich der Draht und bewegt den Betätigungsarm in Richtung M1. Sobald der Draht 1 abgekühlt ist, kann durch Anlegen der Spannung V2 am Memorydraht 2 die Blende 3 wieder in die Ausgangsstellung zurückbewegt werden. Jede Zwischenstellung ist ebenfalls möglich, da die Blende durch ihre Eigenreibung ohne äußeren Krafteinfluss stehen bleibt, sobald der Strom unterbrochen wird.

Da bei einer Verkürzungsrate von ca. 4 % bei einer Erwärmung auf ca. 70° C ein relativ langer Draht notwendig ist, kann er gemäß Figur 2 in Windungen um den Tubus 4 mit der Optik angebracht werden. Dabei ist eine hier nicht dargestellte Führung notwendig, die den sich ausdehnenden Draht führt, und es darf der minimal mögliche Biegeradius des Drahtes nicht unterschritten werden. Die gegeneinander arbeitenden Memorydrähte 1 und 2 greifen einerseits am Betätigungsarm der Blende 3 und andererseits an einem Fixpunkt 5 am Tubus 4 an. Der Pfeil zeigt die optische Achse an.

Nach dem gleichen Prinzip kann auch eine optische Fokussierung betätigt werden.

## Patentansprüche

1. Betätigungsantrieb für eine Optik insbesondere im Bereich Intraoralkamera, **gekennzeichnet durch** zumindest einen an der Optik angreifenden Memorydraht (1, 2) oder Dehnungsdraht und mit einer Einrichtung (V1, V2) zum Erwärmen des Memory- bzw. Dehnungsdrahtes.

2. Betätigungsantrieb nach Anspruch 1, **gekennzeichnet durch** ein elastisches Element zum Rückstellen der Optik bei nachlassender Erwärmung des Memorydrahtes.

3. Betätigungsantrieb nach Anspruch 1, **gekennzeichnet durch** ein elastisches Element zum Einstellen der Optik bei Erwärmung des Dehnungsdrahtes.

4. Betätigungsantrieb nach Anspruch 1, **gekennzeichnet durch** zumindest zwei an der Optik (3) angreifende, gegeneinander arbeitende Memorydrähte (1, 2) und mit einer Einrichtung (V1, V2) zum wechselseitigen Erwärmen dieser Memorydrähte (1, 2).

5. Betätigungsantrieb nach einem der vorhergehenden Ansprüche, **dadurch gekennzeichnet, dass** der Memory- bzw. Dehnungsdraht (1, 2) in Windungen um die Optik (3, 4) angebracht ist.

6. Betätigungsantrieb, nach einem der vorhergehenden Ansprüche, **dadurch gekennzeichnet, dass** die Einrichtung zum Erwärmen des Memory- bzw. Dehnungsdrahts eine elektrische Spannungsquelle (V1, V2) ist.

7. Betätigungsantrieb nach einem der vorhergehenden Ansprüche für den mechanischen Antrieb einer variablen Blende (3) einer Intraoralkamera.

8. Betätigungsantrieb nach einem der vorhergehenden Ansprüche für die Fokussierung der Optik einer Intraoralkamera.

9. Betätigungsantrieb nach Anspruch 7 oder 8 in Verbindung mit Anspruch 5, **dadurch gekennzeichnet, dass** der Memory- bzw. Dehnungsdraht (1, 2) in Windungen um den Tubus (4) mit der Optik zwischen einem Fixpunkt (5) am Tubus und einem Angriffspunkt an der Optik (3) geführt ist.

10. Betätigungsantrieb nach einem der vorhergehenden Ansprüche, **dadurch gekennzeichnet, dass** der Memorydraht (1, 2) einen Durchmesser von ca. 0,1 mm hat und er sich bei einer Erwärmung von 30 - 50° C auf ca. 70° C um ca. 4 % verkürzt.

11. Betätigungseinrichtung nach einem der vorhergehenden Ansprüche, **dadurch gekennzeichnet, dass** der Memorydraht (1, 2) ein Nitinoldraht ist.
